(19) Europäisches Patentamt
European Patent Office
Office européen des brevets

(11) **EP 2 604 700 B1**

(12) **EUROPEAN PATENT SPECIFICATION**

(45) Date of publication and mention
of the grant of the patent:
**22.07.2015 Bulletin 2015/30**

(51) Int Cl.:
***C12Q 1/68*** *(2006.01)*

(21) Application number: **10855744.8**

(22) Date of filing: **13.08.2010**

(86) International application number:
**PCT/CN2010/001230**

(87) International publication number:
**WO 2012/019323 (16.02.2012 Gazette 2012/07)**

(54) **A METHOD FOR ANALYZING CELL CHROMOSOMES**

VERFAHREN ZUR ANALYSE VON ZELLCHROMOSOMEN

PROCÉDÉ D'ANALYSE DES CHROMOSOMES D'UNE CELLULE

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**

(43) Date of publication of application:
**19.06.2013 Bulletin 2013/25**

(73) Proprietor: **BGI Diagnosis Co., Ltd.**
**Guangdong 518083 (CN)**

(72) Inventors:
• **ZHANG, Xiuqing**
**Shenzhen, Guangdong 518083 (CN)**
• **XUAN, Zhaoling**
**Shenzhen, Guangdong 518083 (CN)**
• **CHEN, Fang**
**Shenzhen, Guangdong 518083 (CN)**
• **JIANG, Fuman**
**Shenzhen, Guangdong 518083 (CN)**
• **LIN, Jingrong**
**Shenzhen, Guangdong 518083 (CN)**
• **LI, Peipei**
**Shenzhen, Guangdong 518083 (CN)**

(74) Representative: **Ran, Handong et al**
**RGC Jenkins & Co.**
**26 Caxton Street**
**London SW1H 0RJ (GB)**

(56) References cited:
**WO-A2-03/000919      WO-A2-2007/147079**
**WO-A2-2008/070249      WO-A2-2010/033578**

• **CHIU ROSSA W K ET AL: "Noninvasive prenatal diagnosis of fetal chromosomal aneuploidy by massively parallel genomic sequencing of DNA in maternal plasma", PROCEEDINGS OF THE NATIONAL ACADEMY OF SCIENCES - PNAS, NATIONAL ACADEMY OF SCIENCES, US, vol. 105, no. 51, 23 December 2008 (2008-12-23), pages 20458-20463, XP002620454, ISSN: 0027-8424, DOI: 10.1073/PNAS.0810641105 [retrieved on 2008-12-10] & R. W. K. CHIU ET AL: "Supplementary Information for XP002620454 (Title: Noninvasive prenatal diagnosis of fetal chromosomal aneuploidy by massively parallel genomic sequencing of DNA in maternal plasma)", PROCEEDINGS OF THE NATIONAL ACADEMY OF SCIENCES - PNAS, vol. 105, no. 51, 10 December 2008 (2008-12-10), pages 20458-20463, XP055024153, US ISSN: 0027-8424, DOI: 10.1073/pnas.0810641105**
• **FAN H C ET AL: "Noninvasive diagnosis of fetal aneuploidy by shotgun sequencing DNA from maternal blood", PROCEEDINGS OF THE NATIONAL ACADEMY OF SCIENCES - PNAS, NATIONAL ACADEMY OF SCIENCES, US, vol. 105, no. 42, 21 October 2008 (2008-10-21), pages 16266-16271, XP002613056, ISSN: 0027-8424, DOI: 10.1073/PNAS.0808319105 [retrieved on 2008-10-06]**
• **R. W.K. CHIU ET AL: "Maternal Plasma DNA Analysis with Massively Parallel Sequencing by Ligation for Noninvasive Prenatal Diagnosis of Trisomy 21", CLINICAL CHEMISTRY, vol. 56, no. 3, 1 March 2010 (2010-03-01), pages 459-463, XP055026815, ISSN: 0009-9147, DOI: 10.1373/clinchem.2009.136507**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**(Cont. next page)**

- MARDIS ELAINE R: "Next-generation DNA sequencing methods", ANNUAL REVIEW OF GENOMICS AND HUMAN GENETICS, ANNUAL REVIEWS, US, vol. 9, 1 January 2008 (2008-01-01), pages 387-402, XP002512993, ISSN: 1527-8204, DOI: 10.1146/ANNUREV.GENOM. 9.081307.164359 [retrieved on 2008-06-24]
- SHAN DAN ET AL: "Prenatal Detection of Aneuploidy and Imbalanced Chromosomal Arrangements by Massively Parallel Sequencing", PLOS ONE, vol. 7, no. 2, 28 February 2012 (2012-02-28), page e27835, XP055079017, DOI: 10.1371/journal.pone. 0027835
- Y M. DENNIS LO ET AL.: 'Quantitative Analysis of Fetal DNA in Maternal Plasma and Serum: Implications for Noninvasive Prenatal Diagnosis' AM. J. HUM. GENET. vol. 62, 1998, pages 768 - 775, XP009002852

**Description**

**FIELD OF INVENTION**

This invention relates to a method of analyzing cellular chromosomes, and particularly relates to a method of analyzing the chromosomes of amniotic cells by sequencing.

**BACKGROUND OF INVENTION**

Fetal chromosomal aneuploidy

Fetal chromosomal aneuploidy means a condition that the number of chromosome is not diplontic in a fetal genome. Normally, there are 44 autosomes and 2 sex chromosomes in a human genome in which the male karyotype is (46, XY) and the female karyotype is (46, XX). Fetal chromosomal aneuploidy may refer to the condition of having one more chromosome than a normal diploid fetus, i.e. 47 chromosomes in the fetal genome. Take fetal trisomy 21 for example, compared with a normal diploid fetus, the fetus with trisomy has an extra chromosome 21 with the karyotype of 47, XX (or XY), + 21. Also, fetal chromosomal aneuploidy refers to the condition of missing a chromosome in comparison with the normal diploid fetus, i.e. 45 chromosomes in the fetal genome. For example, the fetus with Turner syndrome with the karyotype of 45, XO misses one chromosome, relative to the normal diploid fetus. Fetal chromosomal aneuploidy also refers to the condition that a part of chromosome is lost, for example, translocated trisomy 21 with the karyotype of 45, XX, der (14; 21)(q10; q10),and Cri du chat syndrome with the karyotype of 46,XX (XY),del (5)(p13).

According to incomplete statistics, the birth rate of fetuses with chromosomal aneuploidy is 1/160 in the world, wherein the birth rate of fetal trisomy 21 (T21, Down syndrome) is 1/800, the birth rate of fetal trisomy 18 (T18, Edwards syndrome) is 1/6000, and the birth rate of trisomy 13 (T13, Patau syndrome) is 1/10000. The development of fetuses with other types of the chromosomal aneuploidy stagnates because some developmental stages could not be accomplished, resulting in clinically unreasoned miscarriage at the early stage of gestation (Deborah A. Driscoll, M.D., and Susan Gross, M.D., Prenatal Screening for Aneuploidy[J]. N Engl J Med 2009; 360:2556-62).

Current situation of culturing amniotic cells

Amniotic cells are epithelial cells floating in the amniotic fluid, which derive from skin, digestive tracts and respiratory tracts of fetus. The procedure of culturing amniotic cells, enriching the number of fetal nucleated cells, preparing chromosomal specimen, and analyzing the fetal chromosomal karyotype is a golden standard of traditionally clinically diagnosing the chromosomal abnormality of fetuses. This technique is reliable, accurate, and enables the observation of abnormalities of chromosome number and structure. Its disadvantages, however, are that it is time-consuming, it takes 10 days to 3 weeks to yield the result, and culturing failure rate is about 1.00% (THEIN AT, ABDEL-FATTAH SA , KYLE PM , et al., An Assessment of the Ase of Interphase FISH with Chromosome Specific Probes as an Alternative to Cytogenetics in Prenatal Diagnosis [J]. PrenatDiagn, 2000, 20(4): 275-280).

The reason of high failure rate of culturing amniotic cells is that the amniotic cells are aging and pyknotic cells, resulting in harder culturing than that of the other tissues (Changjun Ma, Yuania Chen, Peidan Huo; The Culture of Amniotic Cells and the Method of Preparing the Chromosomal Specimen Thereof [J]. Reproduction and Contraception, 1985, 5 (1): 53 - 4). Therefore, successful culturing of amniotic cells plays a critical role in the process of detecting chromosomal aneuploidy. Because of the high requirement of culturing amniotic cells, relatively few viable cells in the amniotic fluid of some of pregnant women, relatively few harvested cells with division phases, and poor chromosomal shape, it is difficult to count and analyze a sufficient number of cells, and detect chromosomes. Furthermore, amniocentesis is a risk with about 2-3% of pregnant women suffering complications, such as uterine contractions, abdominal swelling, tenderness, vaginal bleeding, infection, water leakage, or fetal injury. It would be unacceptable for a pregnant woman if being asked to do second amniocentesis if the culturing of amniotic cells fails or the harvested cells are not sufficient to count and analyze. Moreover, the amniocentesis is generally performed at 16-20 weeks' gestation. Once the culture fails, it is likely that the pregnant woman's gestational period has advanced too far, and thus she may have to undergo cordocentesis with even higher risk. After the culturing of amniotic cells, the analysis of karyotype requires a lot of labor and costs such that many hospitals cannot afford the procedure, causing great difficulties in the clinical spreading and application of amniocentesis.

With the continuous development of sequencing techniques, it is being increasingly applied in the detection and analysis of the chromosome number. Dennis Lo et al. used the peripheral blood of a pregnant woman as experimental material to examine the abnormality of chromosome number by means of massive sequencing based on mathematical statistics methods (Y.M. Dennis Lo, et al., Quantitative Analysis of Fetal DNA in Maternal Plasma and Serum: Implications for Noninvasive Prenatal Diagnosis. Am. J. Hum. Genet. 62:768-775, 1998). But this method cannot completely replace amniocentesis in clinical application, because of some defects occurring in the technique: the cell-free DNAs in plasma are fragmented DNAs, they cannot form a complete genome on their own, the aneuploidy, translocation or mosaicism of the chromosomes other than chromosomes 21, 18, and 13 fails to be detected or have a low detective accuracy. Rossa W. K. Chiu et al. describes, in article "Non-invasive prenatal diagnosis of fetal chromosomal aneuploidy by massively parallel genomic sequencing of DNA in maternal plasma" (PNAS vol. 105, no. 51, 23 December 2008), a locus-independent method of using massively parallel genomic sequencing to quantify maternal plasma DNA sequences for the noninvasive prenatal detection of fetal trisomy 21.

## SUMMARY OF INVENTION

In order to overcome the missed results of detection caused by the sequencing of peripheral blood, and resolve the high failure rate of the culturing of amniotic cells, this invention, in combination with the advantages of the analysis of karyotype by amniocentesis and the method of sequencing the cell-free DNAs in plasma, utilizes a method of detecting chromosomal aneuploidy based on massive sequencing of amniotic cells, including the steps of drawing amniotic cells, isolating DNA, conducting high-throughput sequencing, analyzing the obtained data, and acquiring detection results.

An aspect of the invention is set out in claim 1. In one example, a method of using high-throughput sequencing technique to analyze the chromosomal information of a subject's cells is provided, comprising the steps of:

a. randomly breaking the genomic DNA of the cells, obtaining DNA fragments with a certain size, and sequencing them;

b. strictly aligning the DNA sequences sequenced in step a to the reference sequence of the human genome to obtain the information about the DNA sequences located on a particular chromosome;

c. for the particular chromosome N, determining the total number of the sequences mapped to a sole region of the chromosome, among the above-sequenced DNA sequences, thereby making ChrN% for chromosome N, i.e. ratio of the total number (S1) of the sequences mapped to the sole place of chromosome N, among the above-sequenced DNA sequences, to the total number (S2) of the sequences located on all chromosomes, among the above-sequenced DNA sequences: ChrN%=S1/S2;

d. comparing ChrN% for chromosome N with ChrN% for the corresponding chromosome coming from standard cells to determine whether there exists a difference between the chromosome of the cells and the corresponding one of the standard cells.

The cells may be, for example, amniotic cells, wherein the amniotic cells may be uncultured amniotic cells or cultured amniotic cells. In one embodiment of the invention, to avoid culturing amniotic cells, the amniotic cells are uncultured amniotic cells.

The genomic DNA of the cells may be obtained by traditional methods of isolating DNA, such as salting-out, column chromatography, and SDS, preferably by column chromatography. The so-called column chromatography involves using cell lysis buffer and protease K to treat amniotic cells or tissues to expose naked DNA molecules, making them pass through a silica membrane column capable of binding negatively charged DNA molecules, to which the genomic DNA molecules in the system are reversibly adsorbed, removing the impurities such as proteins or lipids by washing buffers, and diluting by purifying buffers to obtain the DNA of amniotic cells (for more details about specific principles and methods, see the product manuals for product No. 56304 from Qiagen and product DP316 from Tiangen).

The DNA molecules are randomly broken by restriction cleavage, atomization, ultrasound, or HydroShear method. HydroShear method is preferably used (when the solution containing DNA is flowing through a passage with small section, the flowing rate is accelerated, creating a force enough to destruct suddenly the DNA to produce DNA fragments in various sizes depending on the flowing rate and the section area. For more details about specific principles and methods, see product manuals of HydroShear from Life Sciences Wiki). In this way the DNA molecules are broken into fragments with a narrow range of sizes, of which major bands generally range from 200bp to 300bp in size.

The sequencing method adopted in the invention may be the second generation sequencing method such as Illumina/Solexa or ABI/SOLiD. In one embodiment of the invention, the sequencing method is Illumina/Solexa and the resultant sequences are fragments with 35bp in size.

When the DNA molecules to be examined are from multiple samples, each sample may be attached a different tagged sequence *index* so as to be processed during the process of sequencing (Micah Hamady, Jeffrey J Walker, J Kirk Harris et al., Error-correcting Barcoded Primers for Pyrosequencing Hundreds of Samples in Multiplex. Nature Methods, 2008, March, Vol.5 No.3).

The reference sequence of the human genome is produced after the shield of the repeated sequences within the human genome sequence, for example, the latest version of the reference sequence of the human genome in the NCBI database. In one embodiment of the invention, the human genome sequence is the reference sequence of the human genome as shown in version 36 (NCBI Build 36) of NCBI database.

Aligning strictly with the reference sequence of the human genome means that the adopted method of alignment is a fault-intolerant alignment of the sole region located in the reference sequence of the human genome. In one embodiment of the invention, alignment software Eland (a software package provided by Illumina) was used, and the method adopted was an absolute, fault-intolerance alignment.

When the said DNA sequences is a sequence which is able to be located at a sole region of the reference sequence of the human genome, it is defined as sole sequence represented by *Unique reads.* In the invention, for the purpose of avoiding the interference of the repeated sequences, it is needed to remove those DNA sequences located at the regions of tandem repeats and transpositional repeats within the reference sequence of the human genome and merely take into account those DNA sequences, i.e. sole sequences, which may be located at a sole region. Generally, of all the sequenced DNA sequences, about a quarter to a third of DNA sequences are able to be located at a sole region of the genome, i.e. sole sequences. The statistical number of these sole sequences represents the distribution of the DNA sequences on the genomic chromosomes.

Therefore, the sole sequences can assist in the localization of each DNA sequence that is produced by breaking and sequencing the DNA molecules isolated from amniotic cells on a particular chromosome. ChrN% is values produced by normalizing the sole sequences found on different chromosomes, and the values are merely relevant to the size of a particular chromosome rather than the amount of the data being sequenced. Thus the values can be used to analyze the information on individual 46 chromosomes. Therefore, ChrN% is basic value to conduct a chromosomal analysis.

Whether there exists a difference between the number of a particular chromosome in the cellular samples and the standard cells can be determined by drawing a boxplot, wherein a sample for which ChrN% corresponds to an outlier that goes beyond 1.5-3 time or above 3 times the interquartile range is determined to differ from the standard cells in the chromosome number, i.e. aneuploidy.

Determining whether there exists a difference between a particular chromosome respectively in the said cellular samples and in the standard cellular samples may be accomplished by using "z score_ChrN" to indicate the deviation of ChrN% for the said cellular samples from ChrN% for the standard cellular samples.

Specifically, z score_ChrN= (ChrN% for a particular chromosome from detection samples - ChrN% mean (mean_ ChrN%) for the particular chromosome) / ChrN% standard deviation (S.D._ChrN%).

If z score_ChrN is extremely large or small, it means that the deviation of the chromosome number in the cellular detection sample from that of the normal sample is significant. When it reaches a given level of significance, it may be believed that there is an apparent difference between the former number and the latter number.

The average value of ChrN% for a particular chromosome from the standard cellular samples may be determined according to ChrN% for the chromosome from such as at least 10, 20, 30, 50, or 100 standard cellular samples.

The standard cellular samples are the samples of human cells in which the number of the chromosomes is diploid. A normal male cell has 44 autosomes and 2 different sex chromosomes , (46, XY). On the other hand, a normal female cell has 44 autosomes and 2 identical sex chromosomes ,(46, XX).

The ChrN% standard deviation(S.D._ChrN%) for a particular chromosome from the standard cellular samples may be determined according to the ChrN% for the chromosomes, such as at least 10, 20, 30, 50, or 100 standard cellular samples.

In one embodiment of the invention, the standard cellular samples have 20 samples from normal males and 10 samples from normal females, numbered, respectively, with 1, 2, ...,30, in which Nos. 1-20 are the detection samples from normal males, Nos. 21-30 are the detection samples from normal females. The average value of ChrN% (mean_ChrN%) for the standard cellular samples is calculated as follows:

$$\text{Mean\_ChrN\%} = \frac{1}{30} \sum_{m=1}^{30} ChrX\_M)$$

(wherein N represents autosomes 1-22, M represents normal samples Nos. 1-30)

$$\text{Mean\_ChrX\% (male)} = \frac{1}{20} \sum_{m=1}^{20} ChrX\_M$$

(M represents normal male samples Nos. 1-20)

$$\text{Mean\_ChrY\% (male)} = \frac{1}{20} \sum_{m=1}^{20} ChrY\_M$$

(M represents normal male samples Nos. 1-20)

$$\text{Mean\_ChrX\% (female)} = \frac{1}{10} \sum_{m=21}^{30} ChrX\_M$$

(M represents normal female samples Nos. 21-30)

$$\text{Mean\_ChrY\% (female)} = \frac{1}{10} \sum_{m=21}^{30} ChrY\_M$$

(M represents normal female samples Nos. 21-30)

(Note: due to the fluctuation of sequencing and a large number of gaps existing on Y chromosome of the reference sequence, it results in that even for the normal female samples there are a few DNA sequences aligned with Y chromosome. As compared with males, however, the ChrN% for females is much less than that for males. In the examples, the ChrN% for females is around 0.004, whereas the ChrN% for males is around 0.114.)

Based on each ChrN% mean (mean_ ChrN%) for the standard cellular samples obtained by the method described above, the ChrN% standard deviation (S.D._ChrN%) is calculated with the following formula:

$$\text{S.D.\_ChrN\%} = \frac{1}{30} \sum_{m=1}^{30} (ChrN\_M - mean\_ChrN)^2$$

(wherein N represents autosomes 1-22)

$$\text{S.D.\_ChrX\% (male)} = \frac{1}{20} \sum_{m=1}^{20} (ChrX\_M - mean\_ChrX)^2$$

(M represents normal male samples Nos. 1-20)

$$\text{S.D.\_ChrY\% (male)} = \frac{1}{20} \sum_{m=1}^{20} (ChrX\_M - mean\_ChrX)^2$$

(M represents normal male samples Nos. 1-20)

$$\text{S.D.\_ChrX\% (female)} = \frac{1}{20} \sum_{m=1}^{20} (ChrX\_M - mean\_ChrX)^2$$

(M represents normal female samples Nos. 21-30)

$$\text{S.D.\_ChrY\% (female)} = \frac{1}{20} \sum_{m=1}^{20} (ChrX\_M - mean\_ChrX)^2$$

(M represents normal female samples Nos. 21-30)

Since there is a missed X chromosome replaced by Y chromosome among male chromosomes in contrast to female chromosomes, and the whole length of the X chromosome is about 155M, whereas the Y chromosome is about 59M. In detecting these sex chromosomes, it is necessary to establish a set of normal distribution curves concerning ChrX% or ChrY% for different agendas. The most accurate analysis for X chromosome can be obtained from the different agenda-based normal distribution curves.

In one embodiment of the invention, 30 standard cellular samples were selected to conduct the chromosomal analysis. Then a normal distribution curve was established under the requirement of significance level(such as 0.1%) for normal distribution reached in the instance of having a difference between the number of simulated chromosomes and that in standard cells. Thus, the instance of the absolute value of the z score_ChrN being determined to be below 3 was defined by the number of chromosomes being the same as that in the standard cells. On the basis of the results above, then the chromosomes of the detection samples were analyzed as follows:

If the absolute value of the z score amounts to 3, then the samples have a 99.9% probability that they are not among the normally distributed population, i.e. outliers. This means that the chromosome number of the detected cells differs from that of the standard cells, i.e. chromosomal aneuploidy.

If the absolute value of the z score is less than 3, then the samples have a 99.9% probability that they are normal samples, which means that the chromosome number of the detected cells is the same as that of the standard cells.

If the absolute value of the z score is greater than 3, then the samples have a 99.9% probability that they are abnormal samples, which means that the chromosome number of the detected cells differs from that of the standard cells, i.e. chromosomal aneuploidy.

Further, if the absolute value of the z score is greater than 3, for the specific instance of chromosomal aneuploidy occurring in the detected cells, the Z reference value (cutoff value) may be used to determine it. The Z reference value is calculated with the following formula:

$$Z = (mean\_ChrN\,\% \times 0.5 \times X\%) / S.D.\_ChrN\,\%$$

When N represents autosomes, mean_ChrN % and S.D._ChrN % are the means for all of the samples of the standard cells. When N represents sex chromosomes, mean_ChrN % and S.D._ChrN % are the means for the samples of the standard cells of respective agenda;

X may be any integer between, inclusive, -100 and 100, such as -100, -90, -80, -70, -60, -50, -40, -30, -20, -10, 0, 10, 20, 30, 40, 50, 60, 70, 80, 90, 100.

In one embodiment of the invention, when X amounts to 100, it represents that the cellular detection samples have one more chromosome than the standard cells. In one embodiment of the invention, when X amounts to -100, it represents that the cellular detection samples have one less chromosome than the standard cells. When X amounts to 50, it represents that the cellular detection samples have extra half of one chromosome than the standard cells. In one embodiment of the invention, when X amounts to 50, it represents that the cellular detection samples lacks half of one chromosome in comparison with the standard cells.

In calculating the Z reference value for sex chromosomes, mean_ChrN% and S.D._ChrN % are calculated for either female samples or the male samples. That is:

For the male samples, the Z reference value reached may be (mean_ChrN% (male) $\times 0.5 \times$ X%) / S.D._ChrN% (male);

For the female samples, Z reference value reached may be (mean_ChrN% (female) $\times 0.5 \times$ X%) /S.D._ChrN% (female).

When the absolute value of the z score_ChrN is greater than or equal to 3 and reaches the absolute value of the Z reference value, there is a significant difference between the number of a particular chromosome in the detected cells and that in the standard cells equal to X%. For example, when X amounts to 50, it represents that the detected cells have extra half of the particular chromosome compared with the standard cells; when X amounts to 100, it represents that the detected cells have one particular chromosome more than the standard cells.

In one embodiment of the invention, the specific method of analyzing the chromosomes of amniotic cells includes the following steps:

1. DNA isolation and sequencing

A library was built according to the modified Illumina/Solexa standard procedure of building a library after the DNA of amniotic cells was isolated in accordance with the manual from Tiangen Micro Kit. Then adapters for sequencing were added to the both ends of the randomly broken DNA fragments. During the process, a different tagged sequence (index) was attached to each of the samples such that multiple samples could be differentiated in the data obtained from one-time sequencing.

2. Alignment and statistics

After sequencing by using the second generation sequencing technique, known as Illumina/Solexa sequencing (other alternate sequencing methods can also be used to achieve the same or similar effects), the fragmented DNA sequences of a specified size were produced for each sample, which were subjected to alignment strictly with the reference sequence of the human genome. Thus, the information was obtained on the location of the sequences at the corresponding regions of the genome.

Such a restricted alignment was required because it could not be determined from which chromosome a given DNA sequence originated if fault-tolerant alignment or the alignment with multiple regions was allowed. This would be unfavorable for the subsequent analysis of the data.

The total number of the sole sequences located on each chromosome was calculated by taking each chromosome as a unit, thereby making the ChrN% for each chromosome, i.e. ratio of the total number (S1) of the sequences among the above-sequenced DNA sequences, which are located at the sole place of chromosome N, to the total number (S2) of the sequences among the above-sequenced DNA sequences, which are located on all chromosomes: ChrN%=S1/S2.

This is a method of normalization for different samples having a different sequencing amount. Because amniotic cells contain the whole information of 46 chromosomes, theoretically the total number of the sequences located on a given chromosome is directly proportional to the length of the chromosome.

For example, chromosome 1 is the largest chromosome (about 247 M) in the human genome, whereas chromosome 21 is the smallest chromosome (about 47 M) in the human genome, therefore, given a certain total amount of sequencing, the sequencing results from normal diploid amniotic cells are nearly a fixed value. Although in some sequencing and experimental conditions, the ChrN% is not directly proportional to the size of chromosomes, it is usually a fixed value.

3. Data analysis

By a boxplot analysis, it may be directly determined whether the cellular detection samples are likely to differ from the standard cells in the chromosome number. The samples with abnormal values are directly considered as suspect samples, and the others are considered as standard cellular samples. The detailed process is as follows:

In order to help with the data analysis, a boxplot (used for differentiate abnormal values in mathematical statistics) involving the ChrN% produced above is adopted to determine suspect samples. The specific drawing process is as follows:

1) calculating the upper-quartile (75%), median (50%), and lower-quartile (25%);

2) calculating the difference, interquartile range (IQR), between the upper-quartile and lower-quartile,;

3) drawing the upper and lower ranges of a boxplot with the upper limit being the upper-quartile and the lower limit being the lower-quartile, and drawing a horizontal line where the median lies inside the box;

4) values which are 1.5 times greater than the upper-quartile of the interquartile range or 1.5 times less than the lower-quartile of the interquartile range are classified as outliers.

5) beyond the outliers, drawing a horizontal line across the two value points closest to the upper margin and the lower margin, respectively, as a "whisker" of the boxplot.

6) extreme outliers going beyond a distance three times longer than the interquartile range are represented with star points; milder outliers that lie within 1.5-3 times as the distance of the interquartile are represented with hollow points.

The bold line in the middle of the box represents median values, and the upper and lower boarders represent the upper and lower quartiles, respectively. Outliers are defined by the points deviating from 1.5 times the distance between the upper quartile and lower quartile. For example, when the detection samples are standard cellular samples, the ChrN% corresponding to their chromosomes is a fixed value (for example, 1). When the ChrN% corresponding to their chromosomes is 1.5, then the difference can be considered greatly significant, thereby making the samples suspected samples. That is, it is likely that they are samples differed from the standard cells in the chromosome number.

If needed, the ChrN% mean and standard deviation (S.D._ChrN%) may be determined, respectively, by the ChrN% for a particular chromosome corresponding to the standard cellular samples. Then the z score_ChrN for the chromosome from the suspected samples are calculated with the following formula:

$$\text{z score\_ChrN} = (\text{ChrN\% for the particular chromosome from the suspected samples} - \text{ChrN\% mean}) / \text{S.D.\_ChrN\%}$$

If the absolute value of the z score_ChrN is greater than or equal to 3, there is a difference between the number of a particular chromosome in the cellular samples and that in the standard cells.

Further, for the specific instance of an abnormal chromosome number occurring in the cells, reference to the Z reference value (cutoff value) may be used to determine it. Value Z is calculated with the following formula:

$$Z = (\text{mean\_ChrN}\% \times 0.5 \times X\%) / \text{S.D.\_ChrN}\%$$

When N represents autosomes, the mean_ChrN % and S.D._ChrN % are the mean of all the samples of the standard cells. When N represents sex chromosomes, the mean_ChrN % and S.D._ChrN % are the mean of the samples of the standard cells of the respective agenda;

X is assigned to be 50 or 100. Correspondingly, when X is 100, it represents that the cellular detection samples have one more chromosome than the standard cells. When X is 50, it represents that the cellular detection samples have extra half of one chromosome than the standard cells.

In calculating the Z reference value for sex chromosomes, the mean_ChrN% and S.D._ChrN % are the mean for either female samples or male samples, that is:

For the male samples, the Z reference value reached is (mean_ChrN% (male)×0.5×X%) /S.D._ChrN% (male);

For the female samples, the Z reference value reached is (mean_ChrN% (female)×0.5×X%) /S.D._ChrN% (female).

When the absolute value of the z score_ChrN is greater than or equal to 3 and reaches the absolute value of the Z reference value, there is an X% difference between the number of a particular chromosome in the cells and that in the standard cells.

<u>Advantages of the invention</u>

The invention can be used for the analysis of cells, such as amniotic cells. In accordance with the invention, DNA can directly be isolated from amniotic cells to be detected without a subculture, which greatly decreases the difficulties such as uneasy attachment, insufficient number, or failure of culture caused by the culture of amniotic cells.

By using the characteristic of amniotic cells containing the entire genomic information about a fetus, the invention is able to make an analysis of the aneuploidy of all of the chromosomes of the cells, rather than examine only the sex chromosomes X and Y and chromosomes 21, 18, 13.

Besides, though the method of determination involved in the invention, as compared with plasma samples, is also dependent on approximately normal distribution established on standard cellular samples, such dependency on the standard cellular samples is greatly reduced. Additionally, abnormal samples can be directly determined from data abnormalities, assuming sufficient data.

By using the method of the invention, a large number of cellular detection samples can be subjected to batch analysis. Hundreds of thousands of cellular detection samples can be detected at one time, thereby greatly saving labors and costs.

## BRIEF DESCRIPTION OF FIGURES

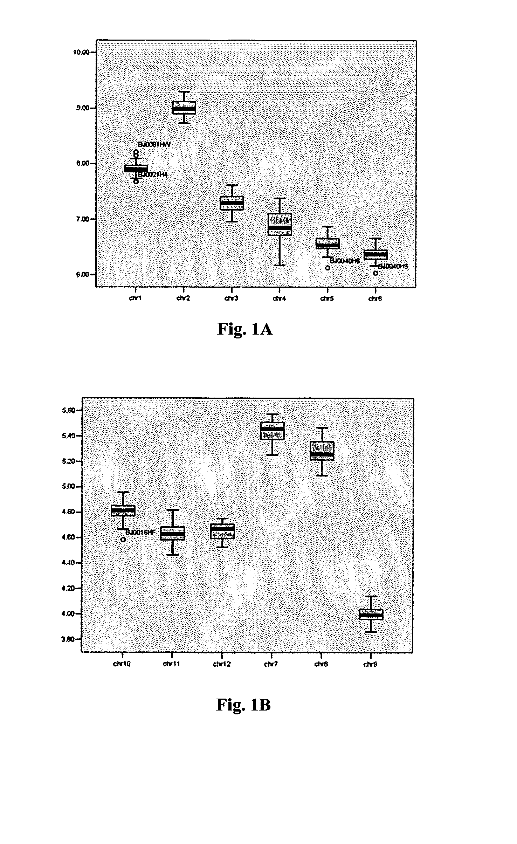

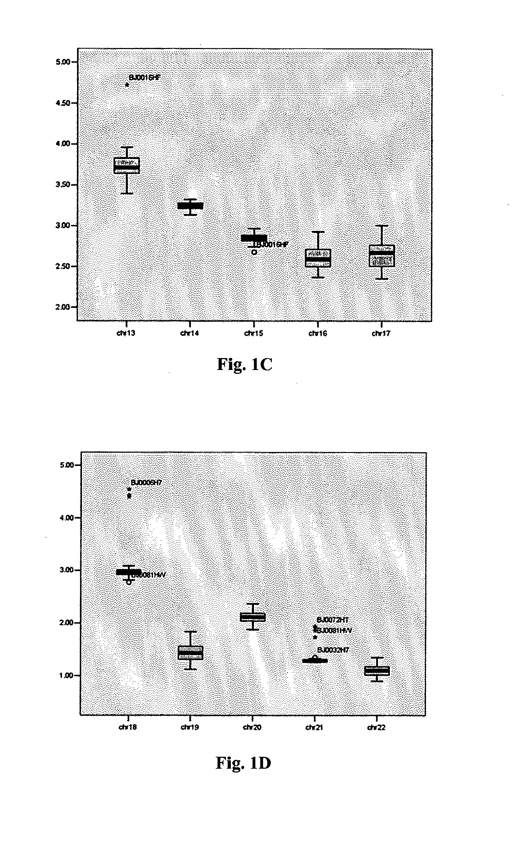

Figure 1 shows a boxplot depicted in accordance with 53 cellular detection samples, in which the abscissa represents the chromosome number, and the ordinate represents the ChrN% value.

Figure 1A shows chromosomes 1-6, figure 1B shows chromosomes 7-12, figure 1C shows chromosomes 13-17, and figure 1D shows chromosomes 18-22.

## DETAILED DESCRIPTION OF THE PREFERRED EMBODIMENTS

The embodiment of the invention will be described in detail in combination with the following examples. A person skilled in the art would appreciate, however, that the following examples are merely intended to make a description of the invention and would not be regarded as the limitation of the scope of the invention. If specific conditions are not specified in the examples, these examples are performed in accordance with commonly used conditions or those advised by manufacturers. If the sources of the reagents or equipment or instruments used in the invention are not specified, such as their manufacturers, all of them are commercially available. The used linkers for sequencing and the tagged sequences *index* come from Multiplexing Sample Preparation Oligonutide Kit provided by Illumina.

In the following parentheses is manufacturers' product number for each of the reagents or kits.

Example 1: Chromosomal analysis of uncultured amniotic cell.

## 1. Isolation and sequencing of DNA

DNA of amniotic cells was isolated according to the procedure of manipulation of a small amount of genome of Tiangen Micro Kit (DP316), and quantitated with Qubit (Invitrogen, the Quant-iT™ dsDNA HS Assay Kit). The total amount of the isolated DNA varied from 100ng to 500ng.

The isolated DNA was either the entire genomic DNA or partially degraded smear-like DNA. A DNA library was built under the standard library-building procedure provided by the modified Illumina/Solexa. Adapters were added to both ends of randomly broken DNA molecules, and attached with different tagged sequence indexes. Then these molecules were hybridized with complementary adapters on the surface of a flow cell, and allowed to be clustered in particular conditions. 36 sequencing cycles were run on an Illumina Genome Analyzer II , producing DNA fragments with 35bp.

Specifically, Diagenode Bioruptor was used to randomly break about 100-500ng of DNA isolated from amniotic cells into 300bp fragments. 100-500ng of initially broken DNA was used to build a library under Illumina/Solexa. See the prior art for a detailed procedure (Illumina/Solexa manual for standard library-building provided by http://www.illumina.com/). The size of the DNA library was determined by way of 2100 Bioanalyzer (Agilent), and the inserted fragments were 300bp. After accurate quantitation by QPCR, sequencing was performed.

In the example, batch sequencing was conducted of 53 DNA samples isolated from amniotic cells according to Cluster Station and GA II x (SE sequencing) officially published by Illumina/Solexa.

## 2. Alignment and statistics

Refer to the prior art (see the manual concerning Pipeline method provided at http://www.illumina.com/), the sequence information obtained in step 1 was subjected to one single Pipeline process, and sequences with low quality were removed, finally resulting in ELAND alignment result against the reference sequence of the human genome of NCBI version 36. Then the number of the sole sequences located on chromosomes was statistically analyzed.

The ChrN% for 22 chromosomes and the X/Y chromosome respectively from 53 samples was calculated and a boxplot (see figure 2) was drawn based on the data. The ChrN% for a particular chromosome N in particular sample M is calculated with the following formula:

Percentage of a particular chromosome in detection sample M, ChrN% = the total number of the sole sequences contained in sample M and located on the corresponding chromosome of the reference sequence through alignment (S1) / the total number of the sole sequences contained in sample M and located on all of the chromosomes of the reference sequence through alignment (S2).

## 3. Data analysis

According to the boxplot drawn in step 2, it was firstly determined whether an outlier existed. That is, as compared with the upper and lower boarders, if a suspected sample deviated far from the point that was 1.5 times the difference between the upper-quartile and the lower-quartile away, it was likely that it differed from the standard samples in chromosome number.

Specifically, the distribution of the boxplot was observed, and 8 suspected samples (sample Nos. P1-P8) were detected. A normal distribution was established by using as standard samples the data concerning 20 normal males and 10 normal females, chosen randomly from the remaining 45 standard cellular samples after the suspected samples were removed. The ChrN% mean (mean_ChrN%) for each chromosome is designated by mean_ChrN% and standard deviation (S.D. _ChrN%) is given in table 1.

Table 1: ChrN%, mean, and standard deviation (S.D.) for each chromosome in standard cells

| Number | Chr1 % | Chr2 % | Chr3 % | Chr4 % | Chr5 % | Chr6 % | Chr7 % | Chr8 % | Chr9 % | Chr1 0% | Chr1 1% | Chr1 2% |
|---|---|---|---|---|---|---|---|---|---|---|---|---|
| 1 | 7.850 | 9.295 | 7.542 | 7.381 | 6.876 | 6.660 | 5.499 | 5.468 | 3.942 | 4.747 | 4.603 | 4.744 |
| 2 | 7.965 | 9.088 | 7.483 | 6.907 | 6.686 | 6.482 | 5.494 | 5.331 | 4.040 | 4.834 | 4.645 | 4.699 |
| 3 | 7.935 | 9.121 | 7.414 | 6.989 | 6.649 | 6.491 | 5.500 | 5.324 | 4.035 | 4.834 | 4.704 | 4.695 |
| 4 | 7.866 | 9.237 | 7.618 | 7.353 | 6.875 | 6.585 | 5.530 | 5.424 | 3.976 | 4.722 | 4.546 | 4.743 |
| 5 | 7.847 | 9.179 | 7.371 | 7.100 | 6.784 | 6.509 | 5.535 | 5.374 | 3.988 | 4.843 | 4.587 | 4.721 |
| 6 | 7.752 | 9.247 | 7.617 | 7.337 | 6.871 | 6.600 | 5.573 | 5.401 | 3.960 | 4.776 | 4.564 | 4.742 |
| 7 | 7.920 | 9.149 | 7.501 | 7.178 | 6.826 | 6.607 | 5.515 | 5.360 | 4.003 | 4.792 | 4.598 | 4.748 |
| 8 | 8.089 | 8.953 | 7.289 | 6.614 | 6.532 | 6.317 | 5.462 | 5.237 | 4.059 | 4.922 | 4.711 | 4.698 |
| 9 | 8.155 | 9.005 | 7.190 | 6.459 | 6.565 | 6.267 | 5.529 | 5.238 | 4.154 | 4.950 | 4.819 | 4.573 |
| 10 | 7.961 | 9.133 | 7.362 | 6.768 | 6.627 | 6.418 | 5.520 | 5.280 | 3.987 | 4.822 | 4.674 | 4.734 |
| 11 | 8.079 | 8.980 | 7.217 | 6.602 | 6.493 | 6.294 | 5.504 | 5.219 | 4.078 | 4.921 | 4.770 | 4.712 |
| 12 | 7.953 | 9.205 | 7.499 | 7.173 | 6.786 | 6.533 | 5.535 | 5.343 | 3.977 | 4.789 | 4.654 | 4.664 |
| 13 | 7.986 | 9.051 | 7.360 | 6.848 | 6.701 | 6.446 | 5.541 | 5.376 | 4.065 | 4.822 | 4.701 | 4.689 |
| 14 | 8.111 | 9.040 | 7.210 | 6.905 | 6.548 | 6.364 | 5.472 | 5.341 | 4.012 | 4.884 | 4.725 | 4.677 |
| 15 | 8.032 | 9.002 | 7.325 | 6.818 | 6.571 | 6.369 | 5.537 | 5.345 | 4.064 | 4.913 | 4.672 | 4.682 |
| 16 | 8.075 | 8.977 | 7.199 | 6.664 | 6.515 | 6.349 | 5.480 | 5.311 | 4.068 | 4.856 | 4.741 | 4.703 |
| 17 | 7.878 | 9.184 | 7.502 | 7.221 | 6.793 | 6.592 | 5.523 | 5.405 | 3.990 | 4.763 | 4.588 | 4.727 |
| 18 | 7.873 | 9.165 | 7.502 | 7.194 | 6.775 | 6.553 | 5.496 | 5.384 | 4.025 | 4.786 | 4.629 | 4.755 |
| 19 | 7.911 | 9.119 | 7.574 | 7.286 | 6.830 | 6.507 | 5.539 | 5.365 | 3.949 | 4.781 | 4.577 | 4.695 |
| 20 | 8.013 | 9.186 | 7.394 | 6.822 | 6.634 | 6.384 | 5.475 | 5.297 | 4.033 | 4.849 | 4.657 | 4.678 |
| 21 | 7.739 | 8.991 | 7.232 | 6.822 | 6.503 | 6.260 | 5.374 | 5.214 | 3.941 | 4.797 | 4.629 | 4.591 |
| 22 | 7.887 | 8.962 | 7.178 | 6.730 | 6.471 | 6.259 | 5.353 | 5.215 | 3.978 | 4.754 | 4.608 | 4.566 |
| 23 | 7.921 | 8.903 | 7.258 | 6.803 | 6.438 | 6.285 | 5.372 | 5.234 | 3.973 | 4.739 | 4.624 | 4.626 |
| 24 | 7.807 | 8.900 | 7.271 | 6.954 | 6.526 | 6.320 | 5.357 | 5.283 | 3.968 | 4.772 | 4.585 | 4.609 |
| 25 | 7.681 | 9.020 | 7.359 | 7.172 | 6.685 | 6.440 | 5.421 | 5.255 | 3.922 | 4.667 | 4.502 | 4.665 |

(continued)

| Number | Chr1 % | Chr2 % | Chr3 % | Chr4 % | Chr5 % | Chr6 % | Chr7 % | Chr8 % | Chr9 % | Chr10 % | Chr11 % | Chr12 % |
|---|---|---|---|---|---|---|---|---|---|---|---|---|
| 26 | 7.892 | 8.827 | 7.205 | 6.728 | 6.529 | 6.305 | 5.363 | 5.123 | 3.914 | 4.847 | 4.616 | 4.557 |
| 27 | 8.071 | 8.730 | 6.993 | 6.186 | 6.159 | 6.034 | 5.267 | 5.150 | 4.044 | 4.919 | 4.790 | 4.556 |
| 28 | 7.878 | 8.771 | 7.059 | 6.452 | 6.418 | 6.210 | 5.318 | 5.208 | 3.962 | 4.912 | 4.593 | 4.613 |
| 29 | 7.803 | 8.985 | 7.335 | 6.934 | 6.613 | 6.382 | 5.428 | 5.268 | 3.892 | 4.744 | 4.510 | 4.658 |
| 30 | 7.992 | 8.818 | 7.126 | 6.540 | 6.363 | 6.167 | 5.329 | 5.089 | 4.028 | 4.820 | 4.689 | 4.548 |
| mean | 7.931 | 9.041 | 7.339 | 6.898 | 6.621 | 6.400 | 5.461 | 5.295 | 4.001 | 4.819 | 4.644 | 4.669 |
| S.D. | 0.116 | 0.146 | 0.163 | 0.300 | 0.172 | 0.148 | 0.082 | 0.091 | 0.057 | 0.069 | 0.078 | 0.065 |

| number | Chr13 % | Chr14 % | Chr15 % | Chr16 % | Chr17 % | Chr18 % | Chr19 % | Chr20 % | Chr21 % | Chr22 % | ChrX % | ChrY % |
|---|---|---|---|---|---|---|---|---|---|---|---|---|
| 1 | 3.917 | 3.294 | 2.762 | 2.401 | 2.380 | 3.051 | 1.120 | 1.966 | 1.295 | 0.920 | 2.163 | 0.126 |
| 2 | 3.743 | 3.279 | 2.885 | 2.595 | 2.650 | 2.980 | 1.431 | 2.148 | 1.296 | 1.088 | 2.128 | 0.123 |
| 3 | 3.770 | 3.268 | 2.914 | 2.565 | 2.602 | 3.027 | 1.434 | 2.112 | 1.270 | 1.073 | 2.154 | 0.119 |
| 4 | 3.944 | 3.254 | 2.801 | 2.416 | 2.402 | 3.080 | 1.140 | 2.000 | 1.279 | 0.914 | 2.170 | 0.126 |
| 5 | 3.880 | 3.234 | 2.866 | 2.545 | 2.576 | 3.049 | 1.354 | 2.064 | 1.275 | 1.050 | 2.154 | 0.112 |
| 6 | 3.909 | 3.322 | 2.792 | 2.393 | 2.389 | 3.089 | 1.139 | 1.966 | 1.309 | 0.910 | 2.217 | 0.127 |
| 7 | 3.868 | 3.305 | 2.833 | 2.451 | 2.481 | 3.002 | 1.266 | 2.033 | 1.327 | 0.942 | 2.165 | 0.129 |
| 8 | 3.585 | 3.258 | 2.943 | 2.800 | 2.863 | 2.996 | 1.685 | 2.262 | 1.296 | 1.223 | 2.091 | 0.116 |
| 9 | 3.590 | 3.205 | 2.926 | 2.860 | 2.960 | 2.879 | 1.669 | 2.271 | 1.339 | 1.240 | 2.046 | 0.111 |
| 10 | 3.714 | 3.305 | 2.873 | 2.677 | 2.767 | 3.001 | 1.539 | 2.168 | 1.307 | 1.154 | 2.085 | 0.122 |
| 11 | 3.638 | 3.303 | 2.927 | 2.787 | 2.872 | 2.947 | 1.687 | 2.244 | 1.294 | 1.275 | 2.041 | 0.115 |
| 12 | 3.823 | 3.285 | 2.829 | 2.493 | 2.504 | 3.012 | 1.323 | 2.068 | 1.293 | 1.025 | 2.116 | 0.120 |
| 13 | 3.734 | 3.282 | 2.819 | 2.676 | 2.670 | 3.002 | 1.479 | 2.100 | 1.299 | 1.097 | 2.135 | 0.121 |
| 14 | 3.647 | 3.274 | 2.894 | 2.746 | 2.758 | 2.939 | 1.501 | 2.256 | 1.308 | 1.147 | 2.120 | 0.121 |
| 15 | 3.720 | 3.262 | 2.891 | 2.663 | 2.730 | 2.976 | 1.551 | 2.176 | 1.287 | 1.164 | 2.122 | 0.130 |
| 16 | 3.584 | 3.269 | 2.970 | 2.767 | 2.868 | 2.965 | 1.667 | 2.186 | 1.270 | 1.277 | 2.125 | 0.114 |
| 17 | 3.864 | 3.246 | 2.827 | 2.475 | 2.476 | 3.036 | 1.228 | 2.024 | 1.297 | 0.963 | 2.277 | 0.120 |

| number | Chr13 % | Chr14 % | Chr15 % | Chr16 % | Chr17 % | Chr18 % | Chr19 % | Chr20 % | Chr2 1% | Chr2 2% | ChrX % | ChrY % |
|---|---|---|---|---|---|---|---|---|---|---|---|---|
| 18 | 3.853 | 3.315 | 2.844 | 2.506 | 2.489 | 3.040 | 1.229 | 2.071 | 1.299 | 0.963 | 2.129 | 0.124 |
| 19 | 3.950 | 3.312 | 2.845 | 2.457 | 2.484 | 3.065 | 1.234 | 2.031 | 1.265 | 0.962 | 2.136 | 0.125 |
| 20 | 3.731 | 3.285 | 2.886 | 2.645 | 2.720 | 2.906 | 1.485 | 2.155 | 1.299 | 1.151 | 2.200 | 0.114 |
| 21 | 3.690 | 3.224 | 2.829 | 2.522 | 2.618 | 2.919 | 1.384 | 2.119 | 1.269 | 1.107 | 4.224 | 0.003 |
| 22 | 3.642 | 3.223 | 2.877 | 2.578 | 2.673 | 2.910 | 1.422 | 2.137 | 1.259 | 1.087 | 4.226 | 0.004 |
| 23 | 3.680 | 3.234 | 2.824 | 2.536 | 2.585 | 2.968 | 1.380 | 2.098 | 1.265 | 1.062 | 4.187 | 0.004 |
| 24 | 3.707 | 3.223 | 2.809 | 2.501 | 2.580 | 2.933 | 1.372 | 2.039 | 1.262 | 1.042 | 4.177 | 0.004 |
| 25 | 3.829 | 3.214 | 2.740 | 2.388 | 2.390 | 2.978 | 1.211 | 1.975 | 1.259 | 0.929 | 4.293 | 0.004 |
| 26 | 3.679 | 3.232 | 2.859 | 2.579 | 2.707 | 2.918 | 1.485 | 2.081 | 1.262 | 1.129 | 4.159 | 0.003 |
| 27 | 3.404 | 3.207 | 2.912 | 2.899 | 2.968 | 2.893 | 1.802 | 2.344 | 1.263 | 1.330 | 4.077 | 0.003 |
| 28 | 3.525 | 3.190 | 2.869 | 2.771 | 2.852 | 2.903 | 1.647 | 2.215 | 1.278 | 1.227 | 4.127 | 0.003 |
| 29 | 3.786 | 3.177 | 2.778 | 2.456 | 2.498 | 2.980 | 1.258 | 2.037 | 1.271 | 0.986 | 4.216 | 0.003 |
| 30 | 3.569 | 3.224 | 2.913 | 2.699 | 2.816 | 2.898 | 1.603 | 2.168 | 1.284 | 1.179 | 4.133 | 0.003 |
| mean | 3.733 | 3.257 | 2.858 | 2.595 | 2.644 | 2.978 | 1.424 | 2.117 | 1.286 | 1.087 | - | - |
| S.D. | 0.135 | 0.040 | 0.055 | 0.147 | 0.175 | 0.060 | 0.187 | 0.099 | 0.021 | 0.120 | - | - |
| mean-Chr X/Y%-M | - | - | - | - | - | - | - | - | - | - | 2.139 | 0.121 |
| S.D.-Chr X/Y%-M | - | - | - | - | - | - | - | - | - | - | 0.055 | 0.006 |
| mean-Chr X/Y%-F | - | - | - | - | - | - | - | - | - | - | 4.182 | 0.003 |
| S.D.-Chr X/Y%-F | - | - | - | - | - | - | - | - | - | - | 0.062 | 0.001 |

Furthermore, in order to examine whether the instance of a half chromosome or an additional chromosome existed in the suspected samples, X was assigned to be 50 or 100 and the corresponding chromosomal Z reference value (cutoff value) was calculated (see table 2):

$$Z = (\text{mean\_ChrN\%} \times 0.5 \times X\%) / \text{S.D.\_ChrN\%}, \text{ wherein N represents chromosomes } 1 \sim 22, X \text{ is } 50 \text{ or } 100.$$

Table 2: determination of the reference Z value (cutoff value) for trisome in the detection cells

| Chromosome number | mean_ChrN% | S.D._ChrN% | Reference Z value | |
|---|---|---|---|---|
| | | | above 50% | above 100% |
| chr1 | 7.9307295 | 0.1159668 | 17.0969881 | 34.1939762 |
| chr2 | 9.0408152 | 0.1458970 | 15.4917815 | 30.9835629 |
| chr3 | 7.3394728 | 0.1633916 | 11.2298783 | 22.4597567 |
| chr4 | 6.8980316 | 0.3000703 | 5.7470125 | 11.4940249 |
| chr5 | 6.6213096 | 0.1717845 | 9.6360687 | 19.2721373 |
| chr6 | 6.3996516 | 0.1482759 | 10.7901077 | 21.5802154 |
| chr7 | 5.4613809 | 0.0819567 | 16.6593481 | 33.3186962 |
| chr8 | 5.2953272 | 0.0912159 | 14.5131676 | 29.0263351 |
| chr9 | 4.0009382 | 0.0572472 | 17.4721938 | 34.9443876 |
| chr10 | 4.8192433 | 0.0693516 | 17.3725054 | 34.7450108 |
| chr11 | 4.6436180 | 0.0780559 | 14.8727280 | 29.7454561 |
| chr12 | 4.6690652 | 0.0647597 | 18.0245772 | 36.0491544 |
| chr13 | 3.7325287 | 0.1346539 | 6.9298575 | 13.8597151 |
| chr14 | 3.2568057 | 0.0398485 | 20.4324240 | 40.8648480 |
| chr15 | 2.8578247 | 0.0553827 | 12.9003599 | 25.8007199 |
| chr16 | 2.5948409 | 0.1465308 | 4.4271266 | 8.8542531 |
| chr17 | 2.6442999 | 0.1753843 | 3.7692929 | 7.5385857 |
| chr18 | 2.9780305 | 0.0598664 | 12.4361514 | 24.8723027 |
| chr19 | 1.4242524 | 0.1868697 | 1.9054083 | 3.8108167 |
| chr20 | 2.1171964 | 0.0985564 | 5.3705200 | 10.7410400 |
| chr21 | 1.2858576 | 0.0205784 | 15.6214179 | 31.2428358 |
| chr22 | 1.0872769 | 0.1204085 | 2.2574758 | 4.5149516 |
| chrX-F | 4.1819271 | 0.0615940 | -16.9737663 | -33.9475326 |
| ChrY-F | 0.0034667 | 0.0006359 | / | / |
| chrX-M | 2.1387665 | 0.0545815 | 9.7962061 | 19.5924123 |
| ChrY-M | 0.1207910 | 0.0055482 | 5.4468013 | 10.8856025 |

The z score_ChrN for each chromosome in the suspected samples was calculated with the following formula:

z score_ChrN =(ChrN% for a given chromosome in the detection samples — mean_ChrN%)/S.D. _ChrN%.

Table 3: The z score_ChrN for each chromosome in the suspected samples

| Chromosome Number | Mean | SD | P1 | P2 | P3 | P4 | P5 | P6 | P7 | P8 |
|---|---|---|---|---|---|---|---|---|---|---|
| Chr1 | 7.931 | 0.116 | -1.566 | 0.349 | -0.274 | 2.466 | -0.537 | -0.733 | -0.028 | -2.100 |
| Chr2 | 9.041 | 0.146 | -0.843 | 0.799 | -1.326 | -1.063 | -0.771 | -0.605 | -0.341 | -0.856 |
| Chr3 | 7.339 | 0.163 | -0.487 | 0.498 | -1.781 | -0.924 | -0.346 | -0.394 | 0.256 | 0.129 |
| Chr4 | 6.898 | 0.300 | 0.238 | 0.988 | -1.045 | -1.548 | -0.469 | -0.177 | 0.151 | 0.774 |
| Chr5 | 6.621 | 0.172 | -0.726 | -0.122 | -1.514 | -1.370 | -0.522 | -0.501 | -0.137 | -0.071 |
| Chr6 | 6.400 | 0.148 | -0.572 | 0.174 | -0.929 | -1.521 | -0.509 | -0.181 | -0.281 | -0.172 |
| Chr7 | 5.461 | 0.082 | -0.582 | -0.312 | -1.889 | -1.166 | -0.674 | 0.073 | -0.292 | -1.247 |
| Chr8 | 5.295 | 0.091 | -0.618 | 0.492 | -2.122 | -2.192 | -0.863 | -0.406 | -1.501 | -0.336 |
| Chr9 | 4.001 | 0.057 | -1.659 | 0.700 | 0.747 | -2.107 | -0.038 | -0.916 | -0.546 | -1.032 |
| Chr10 | 4.819 | 0.069 | -0.644 | -0.051 | -0.385 | 0.131 | -0.174 | -0.849 | -0.997 | -0.712 |
| Chr11 | 4.644 | 0.078 | -2.056 | -0.749 | -0.513 | 1.071 | -0.147 | -0.764 | -1.099 | -2.300 |
| Chr12 | 4.669 | 0.065 | -1.121 | -0.489 | -1.854 | -2.140 | -0.616 | -0.562 | -0.137 | -0.682 |
| Chr13 | 3.733 | 0.135 | -0.163 | 0.544 | -1.460 | -2.573 | -0.515 | -0.102 | 0.514 | 7.393 |
| Chr14 | 3.257 | 0.040 | -1.301 | -1.548 | -1.184 | -1.124 | -0.268 | -1.574 | -0.683 | -0.763 |

| Chromosome Number | Mean | SD | P1 | P2 | P3 | P4 | P5 | P6 | P7 | P8 |
|---|---|---|---|---|---|---|---|---|---|---|
| Chr15 | 2.858 | 0.055 | -1.966 | -1.048 | -0.421 | 0.865 | 0.465 | 0.044 | 1.002 | -0.542 |
| Chr16 | 2.595 | 0.147 | -0.963 | -0.739 | 0.305 | 0.779 | -0.096 | -0.336 | -0.405 | -1.787 |
| Chr17 | 2.644 | 0.175 | -0.913 | -0.766 | 0.394 | 0.846 | 0.278 | -0.236 | -0.561 | -1.736 |
| Chr18 | 2.978 | 0.060 | -0.111 | 0.506 | -2.574 | -0.127 | 23.853 | 26.141 | 24.438 | -0.951 |
| Chr19 | 1.424 | 0.187 | -0.586 | -0.664 | 0.660 | 0.340 | 0.191 | -0.141 | -0.608 | -1.489 |
| Chr20 | 2.117 | 0.099 | -0.907 | -0.697 | 0.211 | 0.210 | -0.002 | -0.018 | -0.659 | -2.396 |
| Chr21 | 1.286 | 0.021 | 27.203 | 31.507 | 29.820 | 21.831 | -0.305 | -1.262 | -0.729 | -0.750 |
| Chr22 | 1.087 | 0.120 | -0.604 | -0.743 | 0.616 | 0.747 | -0.026 | -0.220 | -0.467 | -1.310 |
| ChrX-M | 2.139 | 0.055 | / | 0.650 | / | / | -0.967 | -0.678 | -1.963 | / |
| ChrY-M | 0.121 | 0.006 | / | -1.117 | / | / | -0.551 | 0.215 | -0.197 | / |
| ChrX-F | 4.182 | 0.062 | -1.347 | / | -1.428 | -2.272 | / | / | / | 0.700 |
| ChrY-F | 0.003 | 0.001 | 0.881 | / | -0.741 | 0.616 | / | / | / | -0.303 |

As shown from the analysis above, the suspected samples were 8 in total among the 53 detection samples of amniotic cells, in which, for the chromosomes in each of the suspected samples, 8 abnormalities of chromosome number with the absolute value of a z score_ChrN greater than 3 were detected (see table 3). Specifically, they were:

1) Chr21 for P1, Chr21 for P2, Chr21 for P3, and Chr21 for P4;
2) Chr18 for P5, Chr18 for P6, and Chr18 for P7; and
3) Chr13 for P8.

It was determined by checking the Z value obtained when X =100 in table 2 that the number of chromosome 21 in samples P1-P4 and the number of chromosome 18 in samples P5-P7 were one more than the number of the corresponding chromosomes in the standard cells, respectively, and the number of chromosome 13 in P8 was half one more than the number of the corresponding chromosome in the standard cells. That is, P1-P4 were T21 (Down syndrome), and P5-P7 were T18 (Edwards syndrome), and P8 was mosaic T13 (mosaic Patau syndrome). The results were completely consistent with the traditional analysis results of chromosomal karyotype.

Example 2

An additional 6 samples (Q1-Q6) of amniotic cells were treated and sequenced in the same way as the above to produce data for analysis. The z score_ChrN was calculated on mean_ChrN% and S.D. _ChrN% calculated from 30 standard cellular samples in example 1. 3 positive samples were identified from the 6 samples.

Table 4: The ChrN% of 6 detection samples (Q1-Q6)

|  | Q1 | Q2 | Q3 | Q4 | Q5 | Q6 |
|---|---|---|---|---|---|---|
| Chr1% | 7.900099 | 7.781541 | 7.965013 | 7.937310 | 7.835625 | 7.756449 |
| Chr2% | 9.195581 | 8.969471 | 8.998068 | 9.137041 | 8.836921 | 9.014913 |
| Chr3% | 7.389485 | 7.365766 | 7.389563 | 7.452117 | 7.134356 | 7.378118 |
| Chr4% | 7.090694 | 7.005976 | 6.921334 | 7.112517 | 6.510565 | 7.058824 |
| Chr5% | 6.759707 | 6.600836 | 6.604984 | 6.768853 | 6.357255 | 6.605637 |
| Chr6% | 6.541994 | 6.468799 | 6.461957 | 6.545516 | 6.170975 | 6.376054 |
| Chr7% | 5.562187 | 5.423140 | 5.522745 | 5.521768 | 5.342112 | 5.403700 |
| Chr8% | 5.387074 | 5.344078 | 5.357094 | 5.318220 | 5.176933 | 5.275211 |
| Chr9% | 3.946516 | 3.924984 | 4.061791 | 4.037918 | 4.007161 | 3.958868 |
| Chr10% | 4.831699 | 4.680082 | 4.876470 | 4.845395 | 4.798439 | 4.700673 |
| Chr11% | 4.634992 | 4.541423 | 4.682686 | 4.637077 | 4.603257 | 4.462601 |
| Chr12% | 4.727456 | 4.552861 | 4.734034 | 4.700509 | 4.571935 | 4.594756 |
| Chr13% | 3.871131 | 3.749202 | 3.677764 | 3.875716 | 3.475357 | 3.776552 |
| Chr14% | 3.261377 | 3.247342 | 3.285671 | 3.281681 | 3.238633 | 3.207609 |
| Chr15% | 2.875226 | 2.782605 | 2.926826 | 2.866104 | 2.830466 | 2.757703 |
| Chr16% | 2.516559 | 2.443884 | 2.624248 | 2.524383 | 2.665946 | 2.413713 |
| Chr17% | 2.519897 | 2.481007 | 2.684055 | 2.561488 | 2.725292 | 2.495129 |
| Chr18% | 3.026389 | 2.939323 | 3.027751 | 2.994205 | 2.893438 | 2.985936 |
| Chr19% | 1.291162 | 1.240504 | 1.479644 | 1.317938 | 1.482326 | 1.235699 |
| Chr20% | 2.096249 | 2.063225 | 2.174512 | 2.076472 | 2.173705 | 2.049467 |
| Chr21% | 1.290966 | 1.267192 | 1.297270 | 1.291611 | 1.896099 | 1.297050 |
| Chr22% | 0.992960 | 0.989674 | 1.125718 | 1.017386 | 1.164497 | 0.995698 |
| ChrX% | 2.173990 | 4.134076 | 2.117655 | 2.177186 | 4.104752 | 4.197133 |
| ChrY% | 0.116611 | 0.003010 | 0.003148 | 0.001590 | 0.003956 | 0.002508 |

Table 5: The z score_ChrN for 6 samples calculated from the mean-ChrN% and S.D. _ChrN% of 30 negative samples in example 1

| Chromosome number | mean | SD | Q1 | Q2 | Q3 | Q4 | Q5 | Q6 |
|---|---|---|---|---|---|---|---|---|
| Chr1 | 7.9310 | 0.1160 | -0.2664 | -1.2884 | 0.2932 | 0.0544 | -0.8222 | -1.5048 |
| Chr2 | 9.0410 | 0.1460 | 1.0588 | -0.4899 | -0.2941 | 0.6578 | -1.3978 | -0.1787 |
| Chr3 | 7.3390 | 0.1630 | 0.3097 | 0.1642 | 0.3102 | 0.6940 | -1.2555 | 0.2400 |
| Chr4 | 6.8980 | 0.3000 | 0.6423 | 0.3599 | 0.0778 | 0.7151 | -1.2914 | 0.5361 |
| Chr5 | 6.6210 | 0.1720 | 0.8064 | -0.1172 | -0.0931 | 0.8596 | -1.5334 | -0.0893 |
| Chr6 | 6.4000 | 0.1480 | 0.9594 | 0.4649 | 0.4186 | 0.9832 | -1.5475 | -0.1618 |
| Chr7 | 5.4610 | 0.0820 | 1.2340 | -0.4617 | 0.7530 | 0.7411 | -1.4499 | -0.6988 |
| Chr8 | 5.2950 | 0.0910 | 1.0118 | 0.5393 | 0.6823 | 0.2552 | -1.2974 | -0.2175 |
| Chr9 | 4.0010 | 0.0570 | -0.9559 | -1.3336 | 1.0665 | 0.6477 | 0.1081 | -0.7392 |
| Chr10 | 4.8190 | 0.0690 | 0.1840 | -2.0133 | 0.8329 | 0.3825 | -0.2980 | -1.7149 |
| Chr11 | 4.6440 | 0.0780 | -0.1155 | -1.3151 | 0.4960 | -0.0888 | -0.5223 | -2.3256 |
| Chr12 | 4.6690 | 0.0650 | 0.8993 | -1.7868 | 1.0005 | 0.4848 | -1.4933 | -1.1422 |
| Chr13 | 3.7330 | 0.1350 | 1.0232 | 0.1200 | -0.4092 | 1.0572 | -1.9085 | 0.3226 |
| Chr14 | 3.2570 | 0.0400 | 0.1094 | -0.2414 | 0.7168 | 0.6170 | -0.4592 | -1.2348 |
| Chr15 | 2.8580 | 0.0550 | 0.3132 | -1.3708 | 1.2514 | 0.1473 | -0.5006 | -1.8236 |
| Chr16 | 2.5950 | 0.1470 | -0.5336 | -1.0280 | 0.1990 | -0.4804 | 0.4826 | -1.2332 |
| Chr17 | 2.6440 | 0.1750 | -0.7092 | -0.9314 | 0.2289 | -0.4715 | 0.4645 | -0.8507 |
| Chr18 | 2.9780 | 0.0600 | 0.8065 | -0.6446 | 0.8292 | 0.2701 | -1.4094 | 0.1323 |
| Chr19 | 1.4240 | 0.1870 | -0.7104 | -0.9813 | 0.2976 | -0.5672 | 0.3119 | -1.0070 |
| Chr20 | 2.1170 | 0.0990 | -0.2096 | -0.5432 | 0.5809 | -0.4094 | 0.5728 | -0.6822 |
| Chr21 | 1.2860 | 0.0210 | 0.2365 | -0.8956 | 0.5367 | 0.2672 | 29.0523 | 0.5262 |
| Chr22 | 1.0870 | 0.1200 | -0.7837 | -0.8111 | 0.3226 | -0.5801 | 0.6458 | -0.7609 |
| ChrX-M | 2.1390 | 0.0550 | 0.6362 | / | -0.3881 | 0.6943 | / | / |
| ChrY-M | 0.1210 | 0.0060 | -0.7315 | / | -19.6420 | -19.9016 | / | / |
| ChrX-F | 4.1820 | 0.0620 | / | -0.7730 | -33.2958 | -32.3357 | -1.2459 | 0.2441 |
| ChrY-F | 0.0030 | 0.0010 | / | 0.0100 | -1.41 | -1.41/ | 0.9564 | -0.4919 |

As seen from the results, Q5 had an extra copy of chromosome 21 than the standard cells, which was T21; Q3, Q4 missed one copy of chromosome X, which was 45XO (Turner syndrome). The results were completely consistent with the traditional analysis results of chromosomal karyotype.

Although the examples of the invention have been described in great detail, a person skilled in the art will understand that, according to all of disclosed teachings, a variety of modification and replacement may be made of those details. The whole scope of the invention is defined by attached claims and its equivalent.

## Claims

1. A method of analyzing chromosomal information of sample cells, including the steps of:

    randomly breaking a genome DNA from the sample cells to obtain DNA fragments of a certain size;

sequencing the DNA fragments to obtain DNA sequences;

strictly aligning resulting DNA sequences against a reference sequence of human genome to obtain information about location of the DNA sequences on specific chromosomes;

determining a total number of resulting DNA sequences that are located at unique regions on a particular chromosome N;

calculating a value ChrN% for chromosome N, wherein ChrN% = the total number of resulting DNA sequences located at the unique regions on chromosome N / a total number of resulting DNA sequences located at unique regions on all chromosomes; and

determining whether there is a difference between the number of chromosomes in the sample cells and the number of chromosomes in standard cells based on the calculated value ChrN% and a reference value ChrN% for the standard cells,

wherein the determination is performed by comparing a "z score_ChrN" against a reference Z value,

wherein the z score ChrN = (the calculated ChrN% for the sample cells - a ChrN% mean value) / a ChrN% standard deviation, the ChrN% mean value determined based on ChrN% for at least 10 standard cellular samples, and the ChrN% standard deviation determined based on an average value of ChrN% mean values for at least 10 standard cellular samples,

wherein the reference Z value = (ChrN% mean $\times$ 0.5 $\times$ X%) / ChrN % standard deviation, X being an integer between -100 and 100 inclusive, and

wherein when an absolute value of the z score_ChrN is greater than or equal to 3 and reaches an absolute value of the Z reference value, it is determined that there is an X% difference between the number of chromosomes in the sample cells and that in the standard cells.

2. The method of claim 1, wherein the strict aligning is performed based on fault-intolerant alignment of the unique regions located in the reference sequence of human genome, and wherein the reference sequence of human genome is obtained by shielding repeated sequences within the human genome sequence.

3. The method of claim 1, wherein the determination of whether there is a difference between the number of chromosomes in the sample cells and that in the standard cells is performed by boxplot analysis.

4. The method of claim 3, wherein it is determined that a difference exists if the calculated ChrN% corresponds to an outlier that exceeds an interquartile range by more than 1.5 times.

5. The method of claim 3, wherein it is determined that a difference exists if the calculated ChrN% corresponds to an outlier that exceeds an interquartile range by more than 3 times.

6. The method of claim 1, wherein if an absolute value of the z score_ChrN is greater than or equal to 3, it is determined that there is a difference between the number of chromosomes in the sample cells and that in the standard cells.

7. The method of any preceding claim, wherein the sample cells are amniotic cells, such as cultured or uncultured amniotic cells.

**Patentansprüche**

1. Verfahren zur Analyse von chromosomalen Informationen von Probenzellen, das die folgenden Schritte einschließt:

zufälliges Brechen einer genomischen DNA aus den Probenzellen, um DNA-Fragmente einer bestimmten Größe zu erhalten;

Sequenzieren der DNA-Fragmente, um DNA-Sequenzen zu erhalten;

strenges Alignment der sich ergebenden DNA-Sequenzen mit einer Referenzsequenz von menschlichem Genom, um Informationen über die Lage der DNA-Sequenzen auf spezifischen Chromosomen zu erhalten;

Bestimmen einer Gesamtanzahl der sich ergebenden DNA-Sequenzen, die an einzigartigen Regionen auf einem bestimmten Chromosom N liegen;

Berechnen eines Wertes ChrN% für das Chromosom N, worin ChrN% = die Gesamtanzahl der sich ergebenden DNA-Sequenzen, die an den einzigartigen Regionen auf dem Chromosom N liegen / eine Gesamtanzahl der sich ergebenden DNA-Sequenzen, die an einzigartigen Regionen auf allen Chromosomen liegen; und

Bestimmen, ob eine Differenz vorliegt zwischen der Anzahl an Chromosomen in den Probenzellen und der Anzahl an Chromosomen in Standardzellen, bezogen auf den berechneten Wert ChrN% und einen Referenzwert ChrN% für die Standardzellen,

worin die Bestimmung durch Vergleichen von einem "z score_ChrN" mit einem Referenz-Z-Wert durchgeführt wird,

worin der z score_ChrN = (der berechnete ChrN% für die Probenzellen - einem ChrN% Mittelwert) / eine ChrN% Standardabweichung, wobei der ChrN% Mittelwert basierend auf ChrN% für mindestens 10 Standardzellproben bestimmt wird, und die ChrN% Standardabweichung basierend auf einem Durchschnittswert von ChrN% Mittelwerten für mindestens 10 Standardzellproben bestimmt wird,

worin der Z-Referenzwert = (ChrN% Mittel x 0,5 x X%) / ChrN% Standardabweichung, wobei X eine ganze Zahl zwischen einschließlich -100 und 100 ist, und

worin, wenn ein absoluter Wert des z score_ChrN größer ist als oder gleich 3 ist und einen absoluten Wert des Z-Referenzwerts erreicht, bestimmt wird, dass eine X% Differenz zwischen der Anzahl an Chromosomen in den Probenzellen und der in den Standardzellen vorliegt.

2. Verfahren nach Anspruch 1, worin das strenge Alignment basierend auf fehlerintolerantem Alignment der einzigartigen Regionen, die in der Referenzsequenz von menschlichem Genom liegen, durchgeführt wird und worin die Referenzsequenz von menschlichem Genom durch Abschirmen von sich wiederholenden Sequenzen in der menschlichen Genomsequenz erhalten wird.

3. Verfahren nach Anspruch 1, worin die Bestimmung, ob eine Differenz zwischen der Anzahl an Chromosomen in den Probenzellen und der in den Standardzellen vorliegt, durch Boxplot-Analyse durchgeführt wird.

4. Verfahren nach Anspruch 3, worin bestimmt wird, dass eine Differenz vorliegt, falls der berechnete ChrN% einem Ausreißer entspricht, der einen Quartilabstand um mehr als das 1,5-Fache übersteigt.

5. Verfahren nach Anspruch 3, worin bestimmt wird, dass eine Differenz vorliegt, falls der berechnete ChrN% einem Ausreißer entspricht, der einen Quartilabstand um mehr als das 3-Fache übersteigt.

6. Verfahren nach Anspruch 1, worin, falls ein absoluter Wert des z score_ChrN größer ist als oder gleich 3 ist, bestimmt wird, dass eine Differenz zwischen der Anzahl an Chromosomen in den Probenzellen und der in den Standardzellen vorliegt.

7. Verfahren nach einem vorstehenden Anspruch, worin die Probenzellen Amnionzellen sind, wie zum Beispiel kultivierte oder nicht kultivierte Amnionzellen.

**Revendications**

1. Procédé d'analyse de l'information chromosomique à l'intérieur de cellules échantillons, qui comprend les étapes consistant à :

couper de manière aléatoire l'ADN du génome des cellules échantillons pour obtenir des fragments d'ADN d'une certaine taille ;

séquencer les fragments d'ADN pour obtenir des séquences d'ADN ;

effectuer l'alignement strict des séquences d'ADN obtenues avec une séquence de référence du génome humain pour obtenir une information sur l'emplacement des séquences d'ADN sur des chromosomes spécifiques ;

déterminer le nombre total de séquences d'ADN obtenues qui sont situées au niveau de régions uniques sur un chromosome N particulier ;

calculer une valeur ChrN% pour le chromosome N, où ChrN% = le nombre total de séquences d'ADN obtenues qui sont situées au niveau de régions uniques sur le chromosome N/nombre total de séquences d'ADN obtenues qui sont situées au niveau de régions uniques sur tous les chromosomes ; et

déterminer si une différence existe entre le nombre de chromosomes dans les cellules échantillons et le nombre de chromosomes dans des cellules témoins sur la base de la valeur ChrN% calculée et d'une valeur ChrN% de référence pour les cellules témoins,

dans lequel la détermination est effectuée en comparant un « score z_ChrN » à une valeur de référence Z,

dans lequel le score z_ChrN = (le ChrN% calculé pour les cellules échantillons-une valeur ChrN% moyenne)/un écart type du ChrN%, la valeur ChrN% moyenne étant déterminée sur la base du ChrN% obtenu avec au moins 10 spécimens de cellules témoins et l'écart type du ChrN% étant déterminé sur la base d'une valeur moyenne des valeurs ChrN% moyennes obtenues avec au moins 10 spécimens de cellules témoins,
dans lequel la valeur de référence Z = (moyenne du ChrN% x 0,5 x X %)/écart type du ChrN%, où X est un nombre entier entre -100 et 100 inclus, et
dans lequel si une valeur absolue du score z_ChrN est supérieure ou égale à 3 et atteint une valeur absolue de la valeur de référence Z, il est déterminé qu'une différence de X % existe entre le nombre de chromosomes dans les cellules échantillons et le nombre dans les cellules témoins.

2. Procédé de la revendication 1, dans lequel l'alignement strict est effectué sur la base d'un alignement intolérant aux erreurs des régions uniques situées dans la séquence de référence du génome humain et dans lequel la séquence de référence du génome humain est obtenue en protégeant les séquences répétées au sein de la séquence du génome humain.

3. Procédé de la revendication 1, dans lequel l'étape consistant à déterminer si une différence existe entre le nombre de chromosomes dans les cellules échantillons et le nombre dans les cellules témoins est effectuée par une analyse selon la méthode de la boîte à moustaches.

4. Procédé de la revendication 3, dans lequel il est déterminé qu'une différence existe si le ChrN% calculé correspond à une valeur aberrante qui dépasse de plus de 1,5 fois un intervalle interquartile.

5. Procédé de la revendication 3, dans lequel il est déterminé qu'une différence existe si le ChrN% calculé correspond à une valeur aberrante qui dépasse de plus de 3 fois un intervalle interquartile.

6. Procédé de la revendication 1, dans lequel si une valeur absolue du score z_ChrN est supérieure ou égale à 3, il est déterminé qu'une différence existe entre le nombre de chromosomes dans les cellules échantillons et le nombre dans les cellules témoins.

7. Procédé de l'une quelconque des revendications précédentes, dans lequel les cellules échantillons sont des cellules amniotiques, par exemple des cellules amniotiques en culture ou non.

Fig. 1A

Fig. 1B

**Fig. 1C**

**Fig. 1D**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **DEBORAH A. DRISCOLL ; SUSAN GROSS.** Prenatal Screening for Aneuploidy[J]. *N Engl J Med,* 2009, vol. 360, 2556-62
- **THEIN AT ; ABDEL-FATTAH SA ; KYLE PM et al.** An Assessment of the Ase of Interphase FISH with Chromosome Specific Probes as an Alternative to Cytogenetics in Prenatal Diagnosis [J]. *PrenatDiagn,* 2000, vol. 20 (4), 275-280
- **CHANGJUN MA ; YUANIA CHEN ; PEIDAN HUO.** The Culture of Amniotic Cells and the Method of Preparing the Chromosomal Specimen Thereof [J. *Reproduction and Contraception,* 1985, vol. 5 (1), 53-4

- **Y.M. DENNIS LO et al.** Quantitative Analysis of Fetal DNA in Maternal Plasma and Serum: Implications for Noninvasive Prenatal Diagnosis. *Am. J. Hum. Genet.,* 1998, vol. 62, 768-775
- **ROSSA W. K. CHIU et al.** Non-invasive prenatal diagnosis of fetal chromosomal aneuploidy by massively parallel genomic sequencing of DNA in maternal plasma. *PNAS,* 23 December 2008, vol. 105 (51
- **MICAH HAMADY ; JEFFREY J WALKER ; J KIRK HARRIS et al.** Error-correcting Barcoded Primers for Pyrosequencing Hundreds of Samples in Multiplex. *Nature Methods,* March 2008, vol. 5 (3